# EUROPEAN PATENT APPLICATION

(11) **EP 4 830 637 A2**
(43) Date of publication of application: **28.10.2026**
(21) Application number: 26173593.0
(22) Date of filing: 21.04.2026
(51) Int. Cl.: A61B 6/50, A61B 6/00, A61B 6/04, A61B 10/02, A61B 6/02

(54) **SYSTEM AND METHOD FOR DETERMINING OPTIMIZED BREAST COMPRESSION**

(30) Priority: 21.04.2025 US 202563792041 P
(71) Applicant: GE Precision Healthcare LLC, Waukesha, WI 53188 (US)
(72) Inventor: VANCAMBERG, Laurence, 78300 Poissy (FR); SANCHEZ DE LA ROSA, Ruben, 92160 Antony (FR); LECOMTE, Tiphaine, 78180 Montigny Le Bretonneux (FR); SUTTY, Jerome, 91370 Verrieres le Buisson (FR); POUX, Pierre-Olivier, 78350 Les Loges-en-Josas (FR)
(74) Representative: Kilburn & Strode LLP

(57) **Abstract**

According to one aspect of an exemplary embodiment of the disclosure, an imaging device or system, e.g., a mammography imaging system or device, includes an optimized compression module on the imaging system to provide optimized compression of a breast on the mammography imaging system employing information from one or more sensing device(s) disposed on the mammography imaging system. Examples of the types of sensing devices include force sensors, ultrasound sensing devices, MEMS radar devices and/or time-of-flight (ToF) sensing devices. The information provided by the sensing devices is compared with an optimized compression stop point determined by the optimized compression module for use in controlling movement of various components of the imaging system, e.g., a compression plate, to compress the breast to an optimized position to enhance the quality of images to be obtained of the breast and/or maximizing patient comfort.

## Description

### FIELD OF THE DISCLOSURE

The present disclosure relates generally to mammography imaging systems and devices, and more specifically to systems and methods for compression of the breast prior to mammographic imaging.

### BACKGROUND OF THE DISCLOSURE

Embodiments of the invention relate generally to X-ray medical imaging, and more particularly to devices, systems and methods employed to perform various imaging procedures, such as mammography imaging procedures including but not limited to spectral mammography (SM), such as 2D/3D dual-energy contrast-enhanced (CE) mammography exams, full-field digital mammography (FFDM) or digital breast tomosynthesis (DBT) mammography exams.

With regard to the use of mammography devices to obtain images, the process of obtaining high quality mammographic images from breast tissue requires a technician to position the breast of a patient between one or more paddles that compress the breast in order to immobilize and flatten it during image acquisition. The compression force applied to a breast improves image quality by reducing the thickness of the breast while spreading the breast tissue over a larger area; this facilitates interpretation of obtained imagery since the amount of overlying tissue for structures within the imaged breast is minimized.

Reduction of the breast thickness by compression is also important in managing patient radiation dosage. In general, the thicker the compressed breast, the more x-ray attenuation. Therefore, a higher x-ray dosage is necessary when imaging thicker breast tissue as compared to the dosage required for thinner tissue. While greater compression forces are desirable for obtaining clear images with lower radiation dosages, greater compression forces may contribute to patient pain or discomfort. Such patients may not schedule or may delay any future examinations due to the fear of an uncomfortable procedure, thereby possibly increasing the risk that a serious medical condition may not be detected in a timely fashion.

In many diagnostic mammography imaging devices, such that disclosed in US Patent Application Publication No. US20200060632, entitled *Apparatus And Method For Mammographic Breast Compression,* the entirety of which is expressly incorporated herein by reference for all purposes, the compression of the breast on the mammography imaging device is controlled by the technician using a footswitch with a binary positioning system, i.e., the footswitch is moveable between an "on" position to cause movement of the compression paddle(s) and an "off" position where the compression paddle(s) are stationary. When the footswitch is on the "on' position, the paddle(s) is moved towards the breast under the full operational speed of the motor operably connected to the paddle(s) until contact of the paddle(s) with the breast is detected. Further, either just prior to or after the paddle is moved into initial contact with the patient, the movement of the paddle is controlled in a finer manner by the technician to slowly achieve a desired compression force on the breast for optimal imaging of the breast. In performing this more closely controlled, finer movement of the paddle, the technician employs the footswitch or a fine movement adjustment knob to incrementally move the paddle towards the breast and reach the target compressive force to achieve the desired compressive force on the breast.

The contact between the paddle and the breast is detected in various manners, including the view of the technician seeing the paddle contacting and compressing the breast and/or through the use of various systems such as, for example, force sensors disposed on the paddle or other breast-contacting surface that measure the force applied to the breast to maintain the compression forces below certain predetermined thresholds. Depending on the patient, however, even compression forces below predetermined thresholds may cause pain or discomfort.

However, even though the footswitch and/or knob can be operated by the technician to closely control the movement of the paddle to contact the breast with the desired compressive force, there still remains the issue of accurately determining the force to be exerted on the breast to attempt to minimize any unnecessary pain or discomfort to the patient while assuring proper immobilization and breast tissue spread. In the case of the technologist viewing the breast as it is compressed by the paddle, the estimation of when the compression force provided by the paddle on the breast is sufficient can be subjective at best and depends greatly on the experience level of the technician in the operation of the mammography imaging device.

Additionally, suitable sensing devices that emit wave(s) or beam(s) to obtain breast related information can be employed with the mammography imaging devices. These sensing devices include ultrasound (US) sensing devices, micro-electromechanical systems (MEMS) radar sensing devices, and time of flight (ToF) sensing devices, among others, such as those disclosed in US Patent No. 12,251,257, entitled *System And Method For Analyzing Breast Support Environment,* the entirety of which is expressly incorporated by reference herein for all purposes. The sensing device emits wave(s)/beam(s) to be reflected of a breast that do not interfere with the operation of the imaging system 10 and that do not create a harmful condition with regard to the breast that is contacted by and that reflects the wave(s)/beam(s). Even when the mammography imaging device includes force sensors on the paddle and/or detectors to provide a more objective indication of the compressive force or pressure being exerted on the breast, the determination of when to stop the breast compression is subjective and shall be adapted for each patient.

In certain prior art attempts to address the subjectivity issues presented in the application of an effective compression to the breast to be imaged, different automatic systems and processes have been developed, such that disclosed in PCT Application Publication No. WO 2013/076622, entitled *Individual Monitoring Of Compression Force For Mammographic Examinations,* the entirety of which is expressly incorporated by reference herein for all purposes. In this system, the breast contact area is determined and employed within a pressure-force optimization rule that computes the compression force as a function of the breast contact area for comparison with a compression force limit. Depending on the results of the comparison of the breast contact area with the compression force limit, the system can indicate whether the currently applied compression force is appropriate for the breast under examination.

Another example of a prior art system attempting to address these issues is disclosed in PCT Application Publication No. WO 2018/156017, entitled *Mammography-Apparatus,* the entirety of which is expressly incorporated by reference herein for all purposes. In this system, a desired compression force to be applied to the breast under examination is calculated based on a determination of the total body volume of the breast, such that the system can be operated to apply the calculated compression force to the breast.

However, in each of these prior art attempts to solve the issues regarding the subjectivity of the compression of a breast for mammography imaging processes, the mathematical relationships and/or formulas utilized to determine the compression forces to be applied to the breast focus exclusively on the volume/contact area of the breast. As a result, the outputs of these prior art systems do not account for all relevant parameters regarding the breast being images, such that the calculated compression force for the breast is not optimized for the mammography imaging procedure.

Therefore, with regard to each of the aforementioned shortcomings of prior art mammography imaging systems concerning the variability of compression forces applied to the breast being imaged, the resulting variation in the quality of the images obtained of the breast and the overly simplistic calculations utilized in prior art automated compression force determination systems, it is desirable to develop an improved system and method for the operation of the mammography imaging device that can optimize the compression of the breast in each mammography imaging procedure to provide increased quality images and comfort to the patient being imaged.

### SUMMARY OF THE DISCLOSURE

According to one aspect of an exemplary embodiment of the disclosure, an imaging device or system, e.g., a mammography imaging system, includes an optimized compression module to provide an optimized compression stop point based on compression force values calculated from information from one or more sensing device(s) disposed on the mammography imaging system. The sensing device(s) employed with the optimized compression module provide force and distance information that can be employed to calculate an optimal compression force to be exerted on the breast during a compression procedure using a machine learning model. Examples of the types of sensing devices include ultrasound sensing devices, MEMS radar devices and time-of-flight (ToF) sensing devices. In addition, these types of sensing devices are capable of covering a zone encompassing an expanded area spaced from the emission area of the sensing device, such that a number of different points within the zone can be simultaneously sensed by the sensing device to determine the presence and distance of one or more objects from the sensing device(s). This distance information can be employed by the sensing device or a controller/central processing unit operably connected to the sensing device(s) to determine the relative positions to produce a distance map illustrating the position and shape of any objects sensed within the zone by the sensing device(s). For example, the distance information obtained can provide information regarding deformation of the breast over time during compression. In addition, the position information can be obtained by the sensing devices during the operation of the imaging system in order to determine the rate of change of the positions, i.e., the speed of the object relative to one another for use in controlling movement of various components of the imaging system, e.g., a compression plate.

Further, the information obtained regarding the shape of the object being imaged can also be determined as the shape of the object changes, e.g., the change in shape and/or thickness of a breast upon compression between a compression plate and a detector surface. This information can be used as input by the optimized compression module in addition to the various parameters and information obtained by the sensing devices to provide an output to the mammography imaging system allowing the compression of the breast to be stopped at a point where the compression force optimized the quality of the images to be obtained of the breast and/or the comfort of the patient.

According to still another aspect of an exemplary embodiment of the present disclosure, a mammography imaging system includes a gantry including radiation source, a detector alignable with the radiation source, and a compression plate moveable relative to the detector to secure a patient breast therebetween, a controller operably connected to the gantry to control the operation of the radiation source and detector to generate image data, and to control the operation of the biopsy device in an interventional/biopsy mode for the mammography system, the controller including a central processing unit and interconnected database for processing the image data from the detector, a display operably connected to the controller for presenting information to a user, and a user interface operably connected to the controller to enable user input to the controller, and an optimized compression module operably connected to the controller, the optimized compression module including at least one sensing device and operable to generate compression data regarding the breast disposed on the detector and to guide the movement of the compression plate to compress the breast on the detector.

According to still another aspect of an exemplary embodiment of the present disclosure, a method for determining an optimal compression stop point prior to performing a mammography imaging procedure includes the steps of providing a mammography imaging system having a gantry disposed movably disposed on a support surface and including a radiation source, a detector alignable with the radiation source, the detector having a surface on which a breast to be imaged is adapted to be positioned, and a compression plate disposed on the gantry and movable with respect to the radiation source and the detector, a controller operably connected to the gantry to control the operation of the radiation source and detector to generate image data in an imaging procedure performed by the imaging system, the controller including a central processing unit and interconnected database for processing the image data from the detector to create images, a display operably connected to the controller for presenting information to a user, and a user interface operably connected to the controller to enable user input to the controller and an optimized compression module operably connected to the controller, the optimized compression module including at least one sensing device operable to generate compression data to calculate an optimal compression force or pressure to be exerted on the breast, and to generate prediction of future compression data, e.g., by measuring data at points M(0) to M(n) during a compression and predicting measured data at points M(n+1) to M(stop), positioning the breast on the surface between the radiation source and the detector, operating the at least one sensing device to measure parameters and calculate an optimal compression stop point determined by the optimized compression module based on the measured parameters, and optionally adjusting the operation of one or more components of the mammography imaging system based on the comparison of the actual compression data to the optimal compression stop point or the predicted compression data.

These and other exemplary aspects, features and advantages of the invention will be made apparent from the following detailed description taken together with the drawing figures.

### BRIEF DESCRIPTION OF THE DRAWINGS

The drawings illustrate the best mode currently contemplated of practicing the present invention.

In the drawings:
FIG. 1 is a perspective view of an imaging device in the form of a mammography apparatus for imaging the breast tissue of a patient, in accordance with an embodiment of the disclosure;
FIG. 2 is a diagram of the system of FIG. 1, showing the radiation source of the system in a scanning position, in accordance with an embodiment of the disclosure;
FIG. 3 is a perspective view of the mammography system of FIG. 1 including a pair of sensing devices forming part of an optimized compression module in accordance with an embodiment of the disclosure.
FIG. 4 is a schematic view of the optimized compression module of FIG. 3 performing a laterality determination on the breast of a patient.
FIG. 5 is a perspective view of the mammography system of FIG. 1 including a sensing device forming part of an optimized compression module in accordance with another embodiment of the disclosure.
FIG. 6 is a perspective view of the mammography system of FIG. 1 including a pair of sensing devices forming part of an optimized compression module in accordance with another embodiment of the disclosure.
FIG. 7 is schematic view of a breast shape determination performed by the optimized compression module of FIG. 3 in accordance with another embodiment of the disclosure.
FIG. 8 is schematic view of the determination of locations of a compression plate and breast performed by the optimized compression module of FIG. 3 in accordance with an embodiment of the disclosure.
FIG. 9 is schematic view of the determination of locations of a moving compression plate and breast after initial contact with the breast performed by the optimized compression module of FIG. 3 in accordance with an embodiment of the disclosure.
FIG. 10 is schematic view of the determination of locations of the compression plate and breast at final compression of the breast performed by the optimized compression module of FIG. 3 in accordance with an embodiment of the disclosure.
FIG. 11 is schematic view of the determination of an estimated pressure exerted on the breast at final compression of the breast performed by the optimized compression module of FIG. 3 in accordance with an embodiment of the disclosure.
FIG. 12 is a schematic view of a first embodiment of the method of operation and training of the optimized compression module of FIG. 3.
FIG. 13 is a schematic view of the measured parameters forming the temporal data used in the operation and training of the optimized compression module of FIG. 3.
FIG. 14 is a schematic view of a method of addressing noisy ground truths in the training of the optimized compression module of FIG. 3.
FIG. 15 is a schematic view of a second embodiment of the method of operation and training of the optimized compression module of FIG. 3.
FIG. 16 is a schematic view of a second embodiment of the method of operation and training of the optimized compression module of FIG. 3.
FIG. 17 is a schematic view of a second embodiment of the method of operation and training of the optimized compression module of FIG. 3.
FIG. 18 is a schematic view of a second embodiment of the method of operation and training of the optimized compression module of FIG. 3.
FIG. 19 is a schematic view of a federated and continuous method of training and operation of the optimized compression module of FIG. 3.

### DETAILED DESCRIPTION OF THE DRAWINGS

One or more specific embodiments will be described below. In an effort to provide a concise description of these embodiments, all features of an actual implementation may not be described in the specification. It should be appreciated that in the development of any such actual implementation, as in any engineering or design project, numerous implementation-specific decisions must be made to achieve the developers' specific goals, such as compliance with system-related and business-related constraints, which may vary from one implementation to another. Moreover, it should be appreciated that such a development effort might be complex and time consuming, but would nevertheless be a routine undertaking of design, fabrication, and manufacture for those of ordinary skill having the benefit of this disclosure.

When introducing elements of various embodiments of the present invention, the articles "a," "an," "the," and "said" are intended to mean that there are one or more of the elements. The terms "comprising," "including," and "having" are intended to be inclusive and mean that there may be additional elements other than the listed elements. Furthermore, any numerical examples in the following discussion are intended to be nonlimiting, and thus additional numerical values, ranges, and percentages are within the scope of the disclosed embodiments.

As used herein, "electrically coupled", "electrically connected", and "electrical communication" mean that the referenced elements are directly or indirectly connected such that an electrical current may flow from one to the other. The connection may include a direct conductive connection, i.e., without an intervening capacitive, inductive or active element, an inductive connection, a capacitive connection, and/or any other suitable electrical connection. Intervening components may be present.

Further, while the embodiments disclosed herein are described with respect to a mammography apparatus for the 2-dimensional imaging of breast tissue, it is to be understood that embodiments of the invention may be applicable to other types of imaging devices for both 2-dimensional and 3-dimensional imaging including, for example, fluoroscopy, full-filed digital mammography, digital breast tomosynthesis (DBT) and spectral mammography (single or multi-energy), as well as for imaging procedures for tissue other than breast tissue. Further still, embodiments of the invention may be used to analyze tissue, generally, and are not limited to analyzing human tissue.

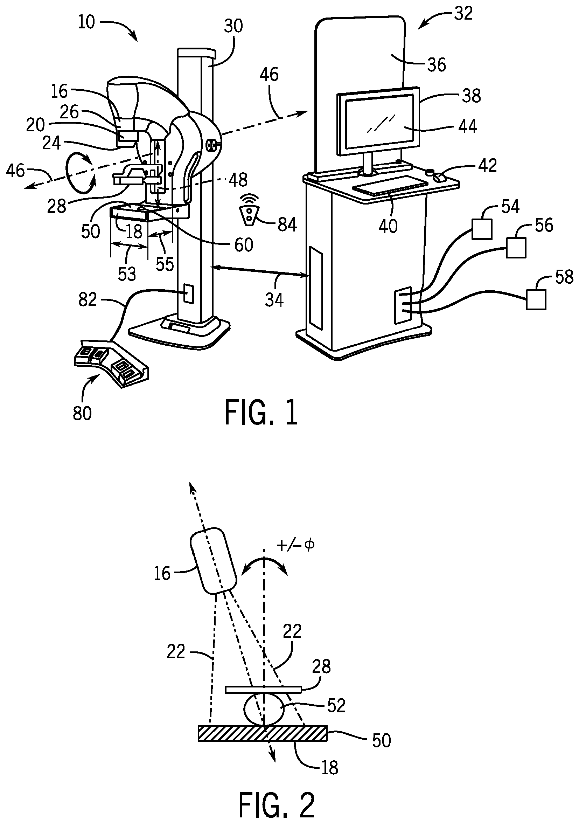

Referring now to FIGS. 1 and 2, the major components of an exemplary imaging system 10 formed as a mammography system 12 for imaging breast tissue according to an embodiment of the invention are shown. The system 10, such that disclosed in US Patent No. US12,251,257, entitled *Apparatus And Method For Analyzing Breast Support Environment,* the entirety of which is expressly incorporated herein by reference for all purposes, includes a radiation source/x-ray source 16, a radiation detector 18, and a collimator 20. The radiation source 16 is movable between a variety of imaging positions relative to the detector 18, and is operative to emit radiation rays 22 (FIG. 2) that are received by the radiation detector 18 to provide an image of a breast 52. In embodiments, the system 10 may include a patient shield 24 mounted to the radiation source 16 via face shield rails 26 to prevent the patient's head from obstructing the radiation rays and protecting the patient from the radiation rays 22.

Referring still further to FIGS. 1 and 2, the system 10 also includes a compression paddle or plate 28 and a support structure 30 to which one or more of the radiation source 16, radiation detector 18, and/or compression plate 28 may be mounted to. In embodiments, the system 10 may further include a controller 32. The controller 32 may be a workstation having at least one processor/central processing unit/computer and a memory device/database that stores information and/or instructions for the operation of the system 10 that are employed by the controller 32, as shown in FIG. 1 or, in other embodiments, the controller 32 may be embedded/integrated into one or more of the various components of the system 10 disclosed above. In embodiments, the controller 32 may be in electrical communication with the radiation source 16, radiation detector 18, and/or the compression plate 28 via a cable 34. As will be appreciated, in embodiments, the connection 34 may be a wireless connection. In embodiments, the controller 32 may include a radiation shield 36 that protects an operator of the system 10 from the radiation rays 22 emitted by the radiation source 16. The controller 32 may further include a display 38, a keyboard 40, mouse 42, and/or other appropriate user input devices that facilitate control of the system 10 via a user interface 44.

As further shown in FIGS. 1 and 2, the radiation source 16, along with the radiation detector 18, forms part of an x-ray system which provides x-ray imagery for the purpose of imaging a body part of a patient, such as breast 52. As stated above, the radiation source 16 emits the radiation rays 22 such that the radiation rays 22 travel from the radiation source 16 to the radiation detector 18. While the radiation rays 22 are discussed herein as being x-rays, it is to be understood that the radiation source 16 may emit other types of electromagnetic rays which can be used to image a patient. The radiation source 16 may be mounted to the support structure 30 such that the radiation source can rotate around an axis 46 in relation to the radiation detector 18, although movement of the radiation source 16 in paths other than rotation about a fixed axis, such as during digital breast tomosynthesis (DBT), are also envisioned. In embodiments, the radiation detector 18 may be configured to rotate or translate within its housing, such as in the directions indicated by arrows 53 and 55.

In the illustrated exemplary embodiment of FIG. 1 the radiation source 16 and the detector 18 are mounted to a gantry 90 that is secured to the support structure 30. The support structure 30 houses a movement mechanism 92 that is operably connected to the gantry 90. The movement mechanism 92 is operable to move the gantry 90 vertically with respect to the support structure 30 in order to position the gantry 90 at the appropriate height to accommodate the dimensions of the patient on which the system 10 is being utilized. The movement mechanism 92 is also operable to rotate the gantry 90 relative to the support structure 30 about the horizontal axis 46 in order to position the gantry 90 rotationally with regard to the patient, as necessary.

The gantry 90 includes a generally C-shaped body 94 with the radiation source 16 at one end and the detector 18 at the opposite end. In this configuration, regardless of the vertical and/or rotational orientation of the gantry 90, such as to position the radiation source 16 and detector 18 relative to the patient breast 52 to obtain x-ray images at various orientations, such as for craniocaudal (CC) or mediolateral oblique (MLO) views, among others, the radiation source 16 is disposed in alignment with the detector 18. In this position, the detector 18 is capable of receiving the x-rays 22 emitted from the radiation source 16 that pass through the portion of the patient, i.e., patient breast 52, located between the radiation source 16 and the detector 18 in order to generate image data for transmission to the control system 32 of the mammography device/system 10 to create/reconstruct a 3D image dataset for viewing by a physician, such as by using DBT, among other known methods.

Additionally, in another embodiment the radiation source 16 can be attached to the gantry 90 to rotate and/or move independently of the gantry 90 and detector 18 in order to enable the radiation source 16 to take x-ray images of the patient breast at various angles relative to the detector 18, e.g., between +/- 60°. The images obtained between these angles for the radiation source 16 can be used either for creation of stereoscopic images in a biopsy procedure using the system 10 or for DBT when operating the system 10 in an imaging mode.

As stated above, the radiation detector 18 receives the radiation rays 22 emitted by the radiation source 16. In embodiments, data regarding the radiation rays 22 received by the radiation detector 18 may be electrically communicated to the controller 32 from the radiation detector 18 via cable/electronic connection 34 such that the controller 32 generates one or more images which may be shown on the display 38 and stored in the memory device.

The compression plate 28 is operative, in response to instruction from the controller 32 or in response to instructions from controller(s) on or near the mammography system 10 or switch controllers 80, to move towards and away from the radiation detector 18 as indicated by arrows/compression axis 48 such that the compression plate 28 flattens and holds a body part, e.g., breast 52, in place against the surface 50 of the radiation detector 18. In this respect, the radiation detector 18 and the surface 50 thereof is referred to herein as a "support plate" that cooperates with the compression plate 28 to compress and clamp a breast of a patient therebetween.

In one exemplary embodiment, in order to maintain the position of the patient breast 52 stationary during the imaging and/or biopsy procedures, the compression plate 28 is attached to a plate or paddle support mechanism 45 located on and/or within the gantry 90 that positions the compression plate 28 directly over and in alignment with the detector 18/support plate and operably connected to the controller 32. The plate support mechanism 45 is operable within the gantry 90 at any rotational or vertical position of the gantry 90 to move the plate 28 in a line either towards or away from the detector 18/support plate. The mechanism 45 can have any of a number of different configurations, but in one exemplary embodiment takes the form of a compression screw mechanism that is operable to move the plate 28 into engagement with the patient breast 52 to exert a predetermined pressure/compression on the breast 52 to retain the breast 52 in a stationary position between the plate 28 and the detector 18/support plate during imaging and/or biopsy procedures.

In operation, in accordance with an embodiment, the breast 52 of the patient may be placed onto the surface 50 of the radiation detector 18. The compression plate 28, under control of the plate support mechanism 45 by the controller 32, moves towards the detector 18 to compress the breast 52 against the surface 50 of the detector 18 such that the breast 52 is immobilized. Movement of the compression plate 28 towards the detector 18 to compress the breast 52 against the support plate/detector 18 defines a compression phase of the system 10. Once a target compression is achieved, movement of the compression plate 28 is halted and the compression plate 28 and the support plate 18 are held in fixed position to clamp the breast 52 therebetween (referred to herein as the clamping phase) so that imaging or procedures, e.g., a biopsy, may be commenced. During an imaging procedure, the radiation source 16 is selectively adjusted such that it is moved/rotated to a first scanning position and scans the breast 52. The radiation detector 18 receives the radiation rays 22 passing through the breast 52 and sends data to the controller 32 which then generates one or more x-ray images of the breast 52. Once imaging is complete, the controller 32 moves the compression plate 28 away from the support plate 18 to free the breast 52.

Referring still further to FIG. 1, in an embodiment, the system 10 may include one or more physiological monitoring or sensor devices 54, 56, 58, 60 communicatively coupled with the controller 32 for monitoring one or more physiological parameters of a patient (and for transmitting physiological parameter data to the controller 32). While FIG.1 illustrates that the sensor devices 54, 56, 58 are connected to the controller 32, in some embodiments, one or more of the sensor devices may be communicatively coupled with the mammography apparatus, without departing from the broader aspects of the invention. The sensor devices may be selected to monitor and/or measure any physiological information of a patient desired, including, but not limited to, diastolic blood pressure, systolic blood pressure, body temperature, blood oxygen level, patient weight, skin conductance, pulse rate, etc. As illustrated in FIG. 1, one or more of the sensor devices, e.g., sensor device 60, may be physically integrated with the compression plate 28 and/or the detector/support plate 18. By incorporating the sensor devices into the support plate 18 or compression plate 28, physiological parameter data of the patient may be acquired and transmitted to the controller 32 without requiring any additional intervention by the system operator.

In an embodiment, the sensor device 60 may be a force sensor for measuring the amount of pressure or compressive force applied to the breast 52. Additional sensors for measuring physiological parameters may be configured to either directly measure or allow the calculation of variables such as force, pressure, blow flow, temperature, rigidity, elasticity, breast size and/or volume, and/or tissue density and could be embedded in compression plate 28 or support plate 18 or attached as part of mammography system 10.

The various sensor devices 54, 56, 58, 60 may be configured to acquire physiological parameter data and/or other sensor data from a patient during system operation. More specifically, physiological parameter data may be acquired continuously or at predetermined time intervals before breast compression and imaging, during the compression phase of the system and/or during the clamping phase of the system. In an embodiment, the physiological parameter data may be acquired continuously or at predetermined time intervals during at least the compression phase. In other embodiments, the physiological parameter data may be acquired continuously or at predetermined time intervals during at least the compression phase and the clamping phase.

Referring once again to FIG. 1, in an embodiment, operation of the system 10 during the compression phase and the clamping phase may be controlled by the patient using switch controls 80. Switch controls 80 are typically connected via a cable/wire 82 to mammography imaging system 10. The controls are also often mirrored on the opposite side of mammography imaging system 10 (not shown). Other controls (not shown) may be present on particular accessories placed either in the paddle/breast support area. In an embodiment, rather than being footswitch controls, the switch controls may be a handheld control unit 84 with a wired, wireless, Bluetooth or other connection with the system 10. In an embodiment, the patient may control the rate of compression and/or pressure or force applied during the compression phase and/or clamping phase using the switch controls 80. A feedback device, e.g. controller 32, may be configured to give feedback information about the image to obtain and may designed such that the feedback information is operatively perceivable by the patient (e.g., through an audible or visual indication). The feedback device, e.g., controller 32, may be configured to provide feedback information to the patient regarding the rate of compression (greater or lower rate of compression) and/or amount of pressure (higher or lower) required to produce an optimal image, in dependence upon the information received from the various sensor devices 54, 56, 58, 60. In this respect, the feedback device informs the patient when compression rate and/or pressure applied is sufficient to obtain a quality image, as determined from a blood pressure or other measurements taken from the patient through sensing devices 54, 56, 58, or 60, before or during the compression and/or clamping phase.

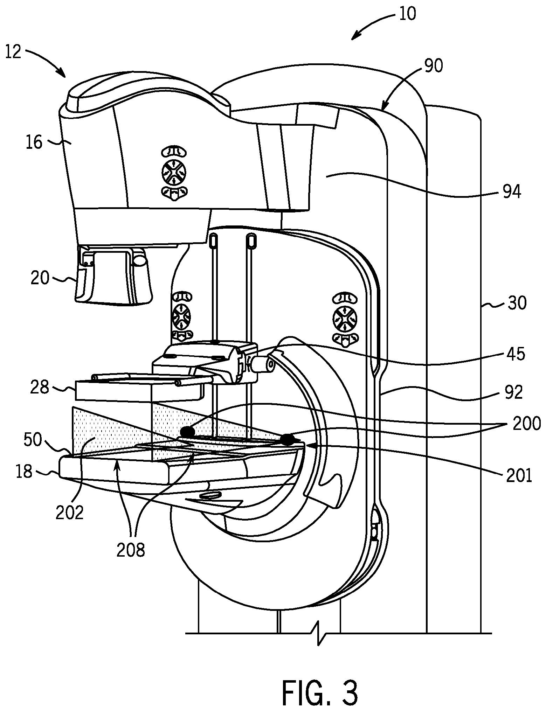

Referring now to FIG. 3, the imaging system 10 further includes one or more of additional sensing devices 200 disposed in one or more positions on the gantry 90 to form a distance and location sensing system 201. The sensing device(s) 200 is capable of emitting one or more waves or beams 202 of sound or radiation that strike an object 204 disposed in the path of the wave(s)/beam(s) 202 emitted from the sensing device 200. Upon striking the object 204, the wave(s)/beam(s) 202 are reflected back to the sensing device 200. The reflected wave(s)/beam(s) 202 provide information to the sensing device 200 regarding the presence, shape, and location of any object 204 in the path of the wave(s)/beam(s) 202 as well as the distance of the object 204 from the sensing device 200. This information is provided by the time elapsed between the emission of the wave(s)/beam(s) 202 from the sensing device 200 and the return of the beam(s) 202 to the sensing device 200 and is analyzed by sensing device 202 and/or the controller 32, which is operably connected to the sensing devices 200, in order to make these determinations. Suitable sensing devices 200 that emit wave(s)/beam(s) 202 to obtain this type of information include ultrasound (US) sensing devices, micro-electromechanical systems (MEMS) radar sensing devices, and time of flight (ToF) sensing devices, among others. In one exemplary embodiment, the sensing device 200 emits wave(s)/beam(s) to be reflected of an object 204 that do not interfere with the operation of the imaging system 10 and that do not create a harmful condition with regard to the object 204 that is contacted by and that reflects the wave(s)/beam(s) 202.

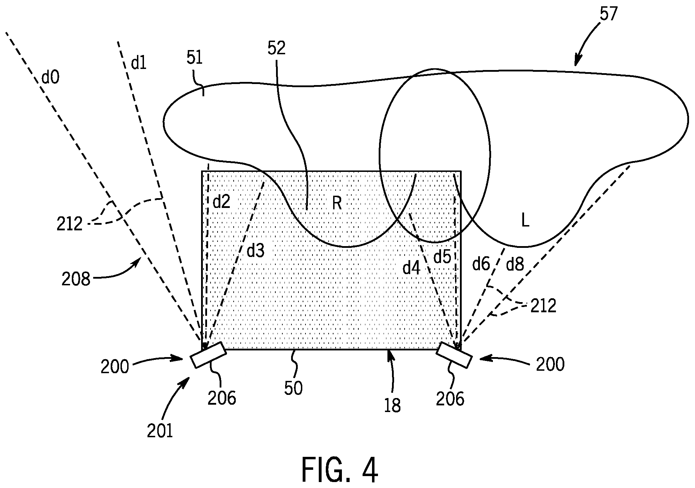

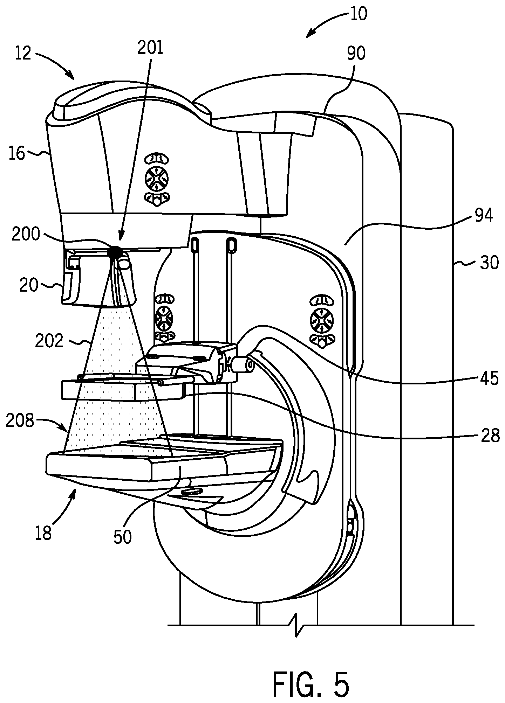

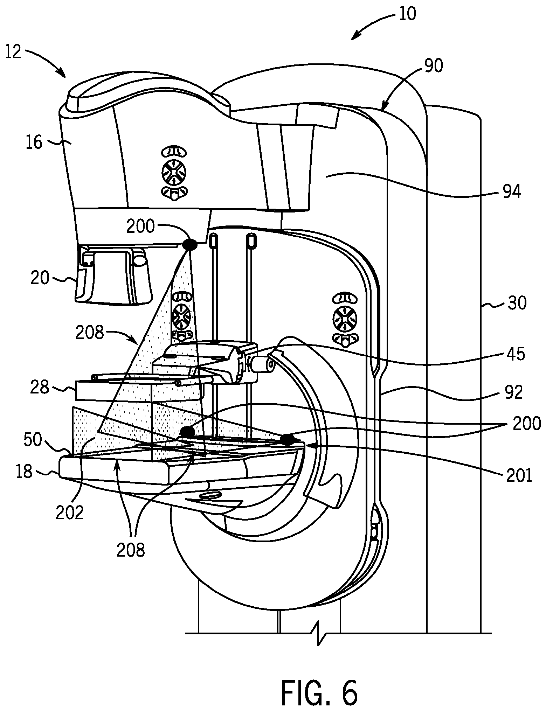

In the illustrated exemplary embodiment of FIGS. 3-5, the imaging system 10 includes at least one (FIG. 5) and in other embodiments a pair (FIGS. 3 and 4) of sensing devices 200 disposed on the detector 18/support plate and oriented to emit the wave(s)/beam(s) 202 towards the object 204. In FIGS. 4 and 5, the object 204 is represented as a breast 52 disposed on the detector 18/support plate to be imaged by the system 10. The sensing devices 202 can also be positioned in different locations or combinations of locations to accommodate for the positions of the sensing devices 200 required to emit wave(s)/beam(s) 202 for determination of the locations of various structures of the imaging system 10 and/or the object 52/204. In particular, as shown in FIGS. 5 and 6, the sensing device(s) 202 can be positioned on or adjacent the radiation source 16 (as the compression plate 28 can be transparent to the waves or radiation emitted by the sensing device(s) 200), on the compression plate 28, and/or at various locations on the gantry 90, either as alternatives or in addition to the placement on the detector 18, in order to provide position information from different perspectives on the locations of various components of the imaging system 10, as well as the patient 57.

With particular reference again to FIGS. 3 and 4, the disposition of the sensing device 200 on the surface 50 of the detector 18 enables the sensing device 200 to emit the wave(s)/beam(s) 202 outwardly towards a breast 52 positioned on the surface 50. In one particular exemplary embodiment, the sensing device(s) 200 can take the form of a ToF sensor 206, such as the ST Microelectronics FlightSense^{™} ToF sensor (https://www.st.com/en/imaging-and-photonics-solutions/time-of-flight-sensors.html). The ToF sensor 206 emits photons into a coverage zone 208 containing a number of individual sectors 212forming the coverage zone 208. The photons are reflected off of a target, such as the breast 52, and return to the ToF sensor 206 for a determination of the distance to the breast 52 from the sensor 206 resulting from the time elapsed from the emission of the photon from the sensor 206 to the sensing of the reflected photon by the sensor 206. The sensor 206 enables multiple photons to be emitted into or across the coverage zone 208 (FIGS. 3-5) in the area targeted by the one or more sensors 206. In one exemplary embodiment, the zone 208 can encompass an 8x8 matrix or grid 210 of individual sectors 212, with each sector 212 within the grid 210 capable of returning a separate location and/or distance determination for any object 52/204 reflecting the photon directed into the particular sector 212, thereby providing a distance map 214 of the object(s) 52/204 detected as being present within the grid 210.

As illustrated in FIG. 4, in order to determine the laterality of the breast 52 disposed on the surface 50 of the detector 18, each of the pair of ToF sensos 206 can emit multiple photons/beams 202 into different sectors 212 d1-d8 in front of the ToF sensors 206. As the patient 57 has only a single breast 52 disposed on the surface 50, the other breast 52 will be disposed to one side of the surface 50. Therefore, the determination of which breast 52 is disposed on the surface 52 can be made through the reflection of the emitted photons/beams 202 off of the breast 52 and the remainder of the patient 53 positioned facing the ToF sensors 206. As shown in the example of FIG. 4, with the right breast 52 positioned on the surface 52, the left side of the patient 57 is disposed in front of the ToF sensor 206 on the right side of the surface 50. Thus, the photons/beams 202 emitted from the ToF sensor 206 covering sectors d5-d8 of the distance map 214 will contact the left side of the patient 57 and be reflected back to the ToF sensor 206. Similarly, the photons/beams 202 emitted from the ToF sensor 206 covering sectors d1-d4 of the distance map 214 will also strike the patient 57 and be reflected back to the ToF sensor 206. However, as the position of the patient 57 does not extend into sectors d1 or d2, the photons/beams 202 directed by the ToF sensor 206 into sectors d1 and d2 will miss the patient 57 and instead will strike and be reflected off of a surface that is an increased distance behind the patient 57. This return delay/increase in the return time for the photons/beams 202 in sectors d1 and d2 can be utilized by the controller 32 to determine that the patient is positioned only within sectors d3-d8 of the resulting distance map 214. As such, with the known position of a breast 52 on the surface 50, and the location of the patient 57 determined within sectors d3-d8, the controller 32 is able to ascertain the laterality of the breast 52 on the surface 50, i.e., that the breast 52 is the right breast 52. Conversely, if the ToF sensors 206 determine that the patient 53 is disposed only in sectors d1-d6, the controller 32 can determine the laterality of the breast 52 on the surface 50 as being the left breast 52.

The ability of the ToF sensors 206 to detect the presence of the body of the patient 57 for determining the laterality of the breast 52 being imaged also enables the ToF sensors 206 to detect parts of the patient 57 other than the breast 52 that may be within the imaging area of the imaging system 10. For example, if an arm 51 of the patient 57 is located too close to the detector 18 and/or is within the FOV of the radiation source 16, the ToF sensors 206 can detect the presence and location of the arm 51 using the photos emitted from the ToF sensors 206 that are reflected back to the ToF sensors 206 from sectors outside of the area of the detector 18/support plate. In particular, as shown in the exemplary embodiment of FIG. 4, the right arm 51 of the patient 57 is located immediately adjacent the detector 18. As such, the photons emitted from the ToF sensor 206 into sectors d1 and/or d2 strike and are reflected by the arm 51 back to the ToF sensor 206. The time taken for the photon(s) to travel to and from the arm 51 is greater than the photos striking the breast 52 on the detector 18, but shorter than those traveling to and from a more remote surface, e.g., a wall behind the patient 57. As a result, the ToF sensors 206 and/or controller 32 can use this information to determine the presence of the arm 51, and the position of the arm 51 relative to the detector 18. With this information, the controller 32 can alert the technician regarding the position of the arm 51Ip, so that the patient 57 can be move the arm 51 out of the FOV of the imaging device 10. The positions of the breast 52 and any other portions of the body of the patient 57, e.g., the arm 51, can additionally be detected through the use of one or more sensing device(s)200 and/or ToF sensors 206 disposed at various positions and/or angles with regard to the detector 18, such as on the support plate 50 and on one or more of the radiation source 16 (FIG. 5) or the paddle/plate 28 (FIG. 6), among other suitable locations.

In addition to the laterality determination for the breast 52, as best shown in FIG. 5, the ToF sensors 206 are positioned in known locations with regard to the surface 50 of the detector 18. Thus, the ToF sensors 206 can emit photons/beams 202 across the breast 52 positioned on the surface 50 to determine a distance map 214 illustrating the shape of the breast 52 on the surface 50, including the position of the nipple 216 which can be utilized to identify the center of the breast 52. With the known position of the surface 50 relative to the sectors d1-d8 scanned by the ToF sensors 206 based on the known relationship between the surface 50 and the positions of the ToF sensors 206, the sensed location of the nipple 216 within the sectors d1-d8 of the distance map 214 can be correlated to a position on the surface 50 by the ToF sensors 206 and/or the controller 32. In this manner, the position of the nipple 216, or any other indication of a center of the breast 52, can be compared with the position of the surface 50 to determine if the breast 52 is properly disposed on the surface 50 prior to compression and/or an imaging procedure. If the controller 32 and/or ToF sensors 206 determine the breast 52 is not properly positioned on the surface 50, and indication can be provided to the technician not only of the improper positioning, but of the forward/back and left/right correction(s) necessary to achieve the proper position for the breast 52 on the surface 50.

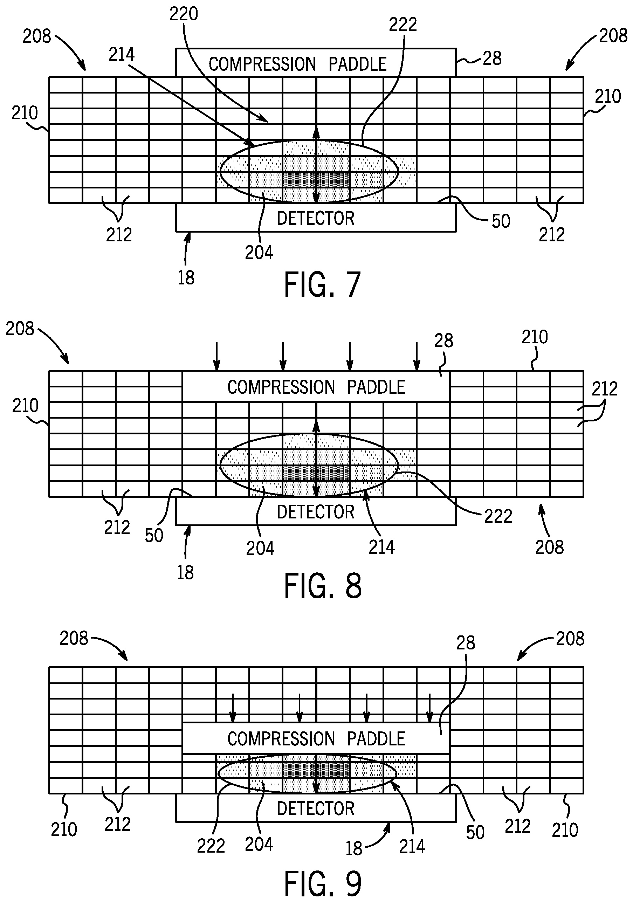

The information obtained by the various sensing device(s) 200, e.g., ToF sensor(s) 206, can additionally be utilized to provide a representation of the shape of the breast 52 disposed on the surface 50 of the detector 18 prior to compression of the breast 52. Referring now to exemplary embodiment illustrated in FIG. 7, the sensing device(s) 202 take the form of a pair of ToF sensor(s) 206 disposed on or adjacent the surface 50 of the detector 18, similar to the configuration of the imaging system of FIG. 3. The ToF sensors 206 each emit photons into adjacent zones 208 each formed of an 8x8 grid 210 of individual sectors 212. The zones 208 cover the exemplary emission area 220 between the compression plate 28 and the detector 18 in order to determine the presence and shape of an object, e.g., a breast 52, disposed on the surface 50 of the detector 18. The travel time of the photons emitted by the ToF sensors 206 into each sector 212 provides the ToF sensors 206 and/or the controller 32 operably connected to the sensors with sufficient information to determine the position of the breast 52 on the surface 50. Further, by employing a suitable matrix segmentation of the distance information provided for each sector 212, the sensors 206 and/or controller 32 can additionally determine the shape of the breast 52 as positioned on the detector 18. As shown in FIG. 7 the matrix segmentation over the individual sectors 212 forming the matrix or grid 210 provides a close estimation for the shape 222 of the breast 52 using information obtained regarding those sectors 212 indicating the presence of the breast 52. In FIG. 7, based on the horizontal orientation of the ToF sensors 206 with regard to the breast 52. The shape 222 determined by the ToF sensors 206 is a front elevational shape of the breast 52. However, with sensing devices 200/ToF sensors 206 disposed at alternative or additional locations on the imaging device/system 10 (see FIGS. 5-6), additional shapes 222 of the breast 52 can be generated from different perspectives, e.g., a top plan shape, right and/or left side elevation shape, etc. in manner similar to that previously described. The additional shapes 222 provided by sensing devices 200/ToF sensors 206 at other locations can be combined with the shape 222 in FIG. 7 to derive a 3D representation of the shape of the breast 52. In addition, the shape 222 of the breast 52 can be continually measured and/or determined in this manner during the compression of the breast 52 to ascertain the changes in the shape 222 of the breast 52 during the entire compression of the breast 52

With this shape information, whether provided by sensing devices 200/ToF sensors 206 enables the controller 32 to match the compression plate 28 to the detected shape 22 of the breast 52. In one manner of doing so, the configuration of the compression plate 28 is input into the imaging system 10, either manually by the technician using one or more of the input devices connected to the imaging system/devoice 10, i.e., the keyboard 40nad/or the mouse 42, or automatically by scanning a suitable identifier (not shown) on the plate 28 containing information about the configuration of the plate 28. Alternatively, the configuration of the plate 28 can be obtained through the use of the sensing device 200/ToF sensors 206, which can scan the plate 28 when disposed within the zones 208 covered by the sensing devices 200/ToF sensors 206, with the distance information obtained by the sensing devices 200/ToF sensors 206 transformed by the controller 32 into a determination of the configuration of the plate 28.

With the information concerning the configuration of the plate 28, the controller 32 can compare the shape 222 of the breast 52 ascertained by the sensing devices 200/ToF sensors 206 to the shape and/or configuration of the plate 28. Should there be a significant difference between the plate configuration and the breast shape 222, the controller 32 can alert the technician of the difference and/or provide information to the technician regarding the plate 28 with the proper shape or configuration most closely matching the shape 22 for the breast 52 to be employed for the imaging and/or biopsy procedure to be performed. In an exemplary embodiment, the information concerning the proper plate 28 to be employed for the imaging procedure can be provided from a database (not shown) of available plates 28 and the specifications/configurations for each plate 28, that can be accessed by the controller 32 for determining a plate 28 having the desired configuration to complement the detected parameters for the breast 52.

According to another exemplary embodiment of the disclosure, referring now to FIGS. 7-10, the location information provided by the sensing device(s) 200/ToF sensor(s) 206 can be employed by the controller 32 to control the movement of the compression plate 28. As shown in FIG. 7, when the breast 52 is initially positioned on the surface 50, the plate 28 is spaced from the surface 50, but is at least partially disposed within the area 220 encompassed within the coverage zone(s) 208 of the sensor(s) 206. A such, the sensor(s) 206 can determine the distance between the breast 52 and the compression plate 28, in addition to the location and shape of the breast 52 on the surface 50.

Looking now at FIGS. 8-10, as the compression plate 28 is lowered towards to the breast 52, the position of the compression plate 28 can be continually determined by the operation of the sensing device(s) 200/ToF sensor(s) 206, which are capable of being operated while the controller 32 moves the compression plate 28 prior to the imaging procedure. In FIG. 8, while the compression plate 28 is moved downwardly towards the breast 52, the position of the compression plate 28, and thus the distance between the plate 28 and the breast 52 is continuously determined, thereby providing the controller 32 with real time information regarding the distance between the plate 28 and the breast 52 as the plate 28 moves toward the breast 52. In this manner the controller 32 can gauge and/or monitor the rate of change of the distance between the compression plate 28 and the breast 52.

Looking now at FIG. 9, as the compression plate 28 begins to contact the breast 52, i.e., when the detected distance between the plate 28 and the breast is 0, or just prior to the contact of the compression plate 28 with the breast 52, the controller 32 can slow down the rate of movement of the plate 28 towards the breast 52. This control of the speed of movement of the compression plate 28 can be done in conjunction with the manual operation of the plate support mechanism 45, where the distance data from the sensing device(s) 200/ToF sensor(s) 206 supplied to the controller 32 enables the controller 32 to provide an automatic braking function with regard to the movement of the compression plate 28. Alternatively, the control of the speed of movement of the plate 28 via the plate support mechanism 45 can be performed independently by the controller 32 using the location/distance data provided to the controller 32 by the sensing device(s) 200/ToF sensor(s) 206.

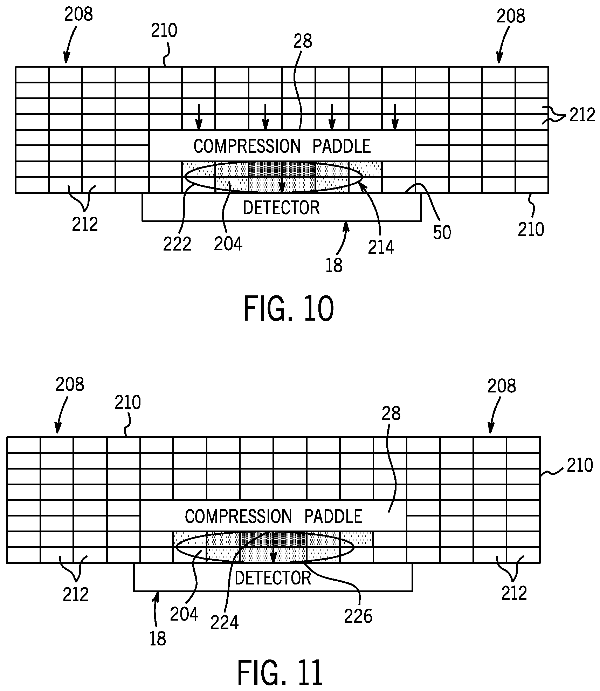

Looking now at FIG. 10, as the plate 28 compresses the breast 52 after initially contacting the breast 52, the controller 32 can further control, i.e., slow the movement of the plate 28, adapt the compression speed from the prediction of the future or predicted compression data or the optimal compression stop point, and ultimately stop all operation of the plate support mechanism 45 and corresponding movement of the plate 28 upon reaching the desired compression for the breast 52 in view of the distance or position information of the plate 28 and breast 52 relative to one another and to the detector 18 as provided to the controller 32 by the sensing device(s) 200/ToF sensor(s)206. Again, similarly to the speed control/slowing of the operation of the plate support mechanism 45 and movement of the plate 28 by the controller 32 prior to contact of the plate 28 with the breast 52, the movement of the plate 28 to compress the breast 52 can be performed automatically by the controller 32 or in conjunction with the manual operation of the plate support mechanism 45.

In addition, similar to the determination of the 3D representation of the breast 52 prior to movement of the compression plate 28 by utilizing distance and/or location data obtained from sensing device(s) 200/sensor(s) 206 located on or adjacent the surface 50, as well as on the plate 28 and/or on the gantry 90 in suitable locations, the distance data from each of the sensing device(s) 200/ToF sensor(s) 206 at different locations on the imaging device 10 can be employed to determine a change in the shape 222 of the breast 52 as the breast 52 is compressed by the plate 28. This determination enables the controller 32 to calculate the changing shape and/or thickness of the breast 52 as it is being compressed by the plate 28, thereby allowing the controller 32 to operate the plate support mechanism 45 to move the plate 28 to a position where the breast 52 is compressed to achieve the optimal thickness for obtaining images of the breast 52. Further, the compression of the breast 52 to the desired thickness can be performed with the controller 32 operating the plate support mechanism 45 in the aforementioned manner to gradually or slowly compress the breast 52 to minimize discomfort to the patient 57 during the compression steps. In connection with the above, it is generally understood that better image quality can be achieved by flattening the breast to a greater degree, by applying a greater compressive force. Increased compressive force, however, is known to cause pain and discomfort for patients. By monitoring the shape 222 of the breast 52 during the compression and clamping phases, the system 10 is able to optimize the compressive force applied and/or the rate of compression to minimize patient discomfort while also ensuring that a minimum level of compression is achieved to ensure that quality images can be obtained. In embodiments, the feedback from the various sensing devices allows for automated, real-time changes in compression based on factors that ensure optimal image quality while at the same time attempting to minimize discomfort experienced during the procedure.

In conjunction with the distance information concerning the changing positions of the compression plate 28 and breast relative to one another and to the detector 18/surface 50, the information provided to the controller 32 by the sensing device(s) 200/ToF sensor(s) 206 as the plate 28 compresses the breast 52 allows for determinations of the pressure being exerted on the breast 52 during compression. In particular, referring now to FIG. 11, the information provided by the sensing device(s) 200/ToF sensor(s) 206 concerning the shape 222 of the breast 52, both prior to and during compression, enables the controller 32 to determine a contact area 224 of the breast 52 with the compression plate 28, and a contact area 226 of the breast 52 with the surface 50 of the detector 18. In FIG. 7, prior to any contact of the compression plate 28 with the breast 52, the breast 52 only has a contact area 226 on the surface 50. Upon initial contact of the plate 28 with the breast 52 (FIG. 8), the breast 52 also creates a contact area 224 with the plate 28. Upon further movement of the plate 28 towards the surface 50 to compress the breast 52 between the plate 28 and the surface 50, the contact areas 224, 226 become larger, as shown in FIGS. 9 and 10.

As the contact areas 224, 226 are enlarged by the movement of the compression plate 28 towards the detector 18/surface 50, the size of the areas 224,226 can be determined by the sensing device(s) 200/ToF sensor(s) 206. In particular, in conjunction with the determination of the shape 222 of the breast 52, the controller 32 can calculate the contact areas 224,226 disposed against the plate 28 and the surface 50. Using these calculated contact areas 224,226, the controller 32 can further compute a pressure that is being exerted on the breast 52 by the compression plate 28 and the surface 50. The determination of the size of the contact areas 224,226 can be performed continuously, i.e., in real-time, throughout the compression of the breast 52 in order to allow the controller 32 to continuously monitor the pressure being exerted on the breast 52. This pressure measurement can also be employed by the controller 32 to provide another manner of controlling the operation of the plate support mechanism 45 to position the plate 28 against the breast 52, such as by moving the plate 28 to compress the breast 52 without exceeding a maximum threshold value or targeted compression or pressure value for the pressure to be exerted on the breast 52, such as one or more predicted compression data point and/or an optimal compression stop point provided by an optimized compression module 300, to be described.

As the controller 32 can be configured to determine the pressure on the breast 52 during compression utilizing only the distance information provided by the sensing device(s) 200/ToF sensor(s) 206, the need for a separate force or pressure sensor 60 on the surface 50 is negated. However, in certain embodiments where the force sensor 60 is already part of an imaging system/device 10 being modified to include the sensing device(s) 200/ToF sensor(s) 206 and accompanying instructions to be stored in memory on the imaging system 10 for the operation of the controller 32 using the distance information from the sensing device(s) 200/ToF sensor(s) 206, the force sensor 60 can be included as a separate manner for the determination of the pressure exerted on the breast 52.

InI operation, regarding the various embodiments discussed previously concerning the construction and operation of the distance and location sensing system 201, the controller 32 is configured to control movement of the compression plate 28 toward the support plate 18 to flatten the breast 52 during the compression phase, and to clamp the breast 52 during the clamping phase, as discussed above. The controller 32 is also configured to control movement of the compression plate 28 to adjust at least one of a rate of compression and/or a pressure or compressive force applied to the breast 52 in response to the physiological parameter data acquired from the patient. For example, in an embodiment, the controller 32 is configured to vary the at least one of the rate of compression and/or the pressure applied to the breast 52 during the compression phase and/or clamping phase based on measurements provided to the controller 32 by the distance or location sensing system 201 regarding of at least one of the shape 222 of the breast 52, the thickness of the breast 52, and/or a computes pressure exerted on the breast 52 taken in a continuous or real-time manner during the compression phase and/or the clamping phase.

In particular, in embodiments where certain distance measurement(s) are obtained via the distance and location sensing system 201 continuously during the compression phase and the clamping phase, the controller 32 may be configured to vary at least one of the rate of compression and/or the pressure applied in real-time, as practicable, as any one or more of these measured distances and associated parameters change. As used herein, "real-time" refers to a level of responsiveness that a user senses as sufficiently immediate, or that enables the controller (or other processor) to keep up with an external process, e.g., compression, pressure, etc. As used herein, "rate of compression" refers to the change in compressive force applied to the breast in relation to elapsed time. In an embodiment, at least one of the rate of compression and/or the pressure applied may be varied in real-time during the compression phase, and a target pressure has been reached for optimal image acquisition, the clamping phase may be initiated and the plates held in static position during the scan (2D, DBT, multi-energy, etc.). This ensures that the object being imaged remains in the same position during the scan.

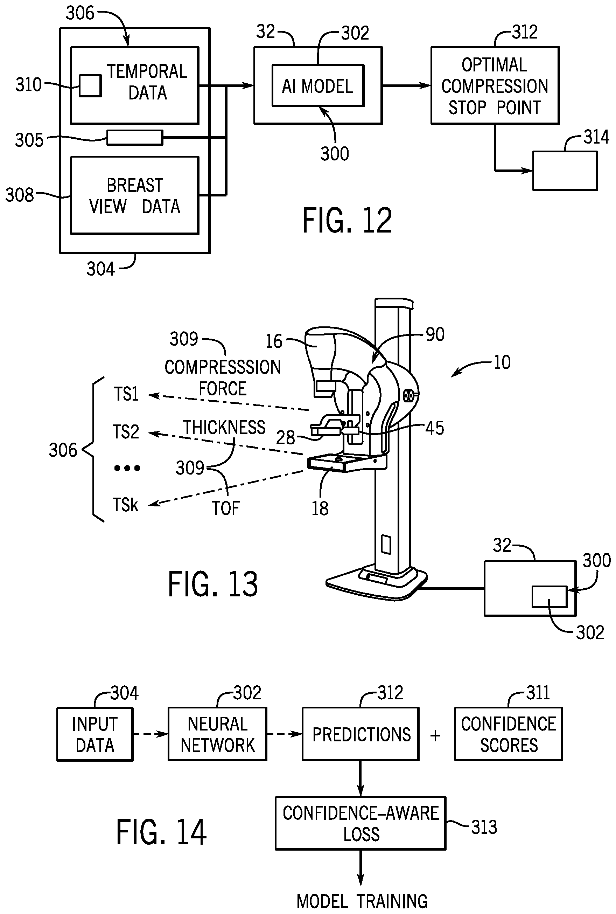

To enable the controller 32 to effectively operate and move the compression plate 28 to compress the breast 52 against the detector 18 to obtain optimized images of the breast 52, referring now to the exemplary embodiment of FIG. 12 the controller 32 includes a compression optimization module 300. The compression optimization module 300 in one embodiment is formed as a part of the controller 32 and is an artificial intelligence that can take the form of a machine learning (ML) model 302 trained to provide predicted compression data, such as in the form of a series of one or more predicted compression data points, e.g., a predicted curve of breast thickness versus force, and/or an optimized compression stop point via the compression plate 28 on a breast 52 to be imaged using the mammography imaging system 10. The ML model 302 is trained to analyze various measured parameters 309 of the mammography imaging system 10 and the breast 52 during a breast compression/procedure, such as the force applied to the breast 52 by the compression plate 28 against the detector 18 in conjunction with the profile, shape and/or thickness of the breast 52 prior to, during and/or after compression, among other measureable parameters 309, in order to output one or more of a series of predicted compression data points regarding the breast compression being performed to and an optimal stop point for the compression of the breast 52 to achieve the desired image quality for the images obtained of the breast 52.

To enable the ML model 302 to accomplish this task in preparation for an imaging procedure, the ML model 302 is trained on one or more training datasets 304. The training dataset 304 constitutes the information to be provided to the optimized compression module 300/ML model 302 when in use after training, such that the methods of training and operation of the optimized compression module 300/ML model 302 utilize highly similar inputs. The training data set 304 can include patient information 305 regarding the demographic(s) and general health condition(s) of the patient being imaged and includes temporal series or temporal data 306 from breast compressions performed prior to mammography imaging procedures. The temporal data 306 includes parameters 309 to be measured/determined by the ML model 302 and recorded versus elapsed time during the compression of a breast 52. As shown in the exemplary embodiment of FIG. 13, these measured parameters 309 include but are not limited to procedure-specific parameters for the mammography imaging system 10, including but not limited to the positions of the compression plate, the breast and the detector relative to each other compute at least one of a breast shape, a 3D breast image, a speed of movement of the compression plate, a pressure or compression force exerted on the breast, a thickness of the breast 52, the profile and/or shape of the breast 52, the tilt angle of the compression plate 28, and/or any operation of a switch 80 by the technologist or patient to control and/or stop the breast compression, and combinations thereof, among others, with at least the compression force and breast thickness being preferred, that are obtained over time during the breast compression procedure. The parameters 309 recorded and stored in the temporal data 306 are obtained from values determined by various sensing devices and controllers operated on the mammography imaging system 10 during compression of the breast 52 prior to performing a mammography imaging procedure. Examples of these types of sensing devices include but are not limited to the sensing device(s) 200/ToF sensor(s) 206, the force sensor 60, as described above and employed to determine the various parameters 309 during the compression of the breast 52. Alternatively or in some combination with the above, the training dataset 304 can comprise raw distance/location data or information concerning the returns received by the ToF sensors 206 in the adjacent zones 208 formed of grids 210 of individual sectors 212 covering the exemplary emission area 220 for the mammography imaging system 10. Using this raw distance information within the training dataset 304, the ML module 302 can be trained to determine one or more of the presence of an object, e.g., a breast 52, the compression plate 28, and/or the surface 50 of the detector 18 within the emission area 220, the positions of the breast 52, the compression plate 18, and the surface 50 relative to one another within the emission area 220, and, such as by employing a suitable matrix segmentation of the distance information provided for each sector 212, the change(s) of the shape and/or position of the breast 52 and the compression plate 28 relative to the surface 50 of the detector 18, along with the ground truth 310 of the technologist and/or patient-controlled stop point.

Included in the dataset 304 in association with the temporal data 306 is image view data 308 concerning the type of image being obtained by the mammography imaging system 10, e.g., a craniocaudal (CC) view, a mediolateral oblique (MLO) view, etc. Additionally, the image view data 308 can include data corresponding to the determined image quality of the resulting image obtained from the particular mammography imaging procedure.

Also, included as a component of the training dataset 304 are ground truth(s) 310 associated with the temporal data 306 and providing information regarding the compression stop point for the breast compressions contained within the temporal data 306. The ground truth 310 can be designated as the stop point as determined by the technician performing the breast compression, with all final values from the various sensing devices 60, 200, 206, e.g., the final compression force, the final compression thickness, the final breast profile, etc., tagged as corresponding to the stop point for the associated compression.. Alternatively, alone or in combination with the technician stop, the ground truth 310 can be designated as the stop point selected by the patient when the mammography imaging system 10 provides the patient with the switch controls 80 that can be used by the patient to halt the compression of the breast 52 when the patient begins to experience significant discomfort or pain from the compression, thus primarily focusing on patient comfort.

When the ground truth 310 is noisy or contains errors or inconsistencies, as illustrated in the exemplary embodiment of FIG. 14, it is possible to add a confidence score or label 311 on the ground truth 310 that is based upon one or more of the patient experience from the breast compression, e.g., an indication on a pain scale for the breast compression, a level of expertise of the technician that performed the breast compression and selected the stop point, image quality metrics for the image resulting from the breast compression, e.g., if the image quality is bad, e.g., the image is blurred, or there is a fold in the compressed breast the data associated with that breast compression could be discarded or given a low level of confidence. With the confidence label, the training of the ML model 302, DL/LSTM model 302' or GNN 302" can employ a weighted loss or confidence aware loss 313 associated with the ground truth 310.

Using the training dataset 304 having the temporal data 306, as well as the optional patient data 305, associated view data 308 and selected ground truth(s) 310, the ML model 302 can be trained and subsequently operated to be able to predict one or more of a series of predicted compression data points regarding the further compression of the breast 52 and the associated optimal compression stop point for the compression of the breast prior to a mammography imaging procedure, e.g., the optimal pressure value and/or compression force value or signal associated with the optimal compression stop point, to obtain the optimal thickness to maximize image quality from the desired view associated with the current mammography imaging procedure and/or minimize pain. The output 312 of this prediction made by the trained ML model 302 analyzes the changes in the measured parameters 309 during the compression of a breast 52 by the mammography imaging system 10 and the selected view, e.g., CC view or MLO view, to be obtained after completing the compression of the breast 52, in comparison with determined relationship(s) of the parameters 309 obtained by the ML model 302 from the training dataset(s) 304 to determine the probability of whether stopping compression at the current configuration for the mammography imaging system 10, i.e., the position of the compression plate 28 relative to the detector 18, would match the optimal compression stop point to achieve the optimal image quality for the image view being obtained, i.e., CC or MLO.

As a result of this output 312, referring back to FIG. 12, after determining one or more of a series of predicted compression data points and/or the optimal compression stop point, the ML model 302 can provide various indications 314 to the technician and/or patient regarding the optimal compression stop point, which can take the form of a visual indicator(s) on the display 38, an audible indicator(s) through a speaker (not shown) operably connected to the controller 32, a haptic indicator(s) disposed within a switch controller 80, and combinations there, among others. For example, in one embodiment the indication 314 can take the form of an indication of how far the level of compression currently applied to the breast 52 is from the optimal stop point determined by the ML model 302. This indication 314 can be based off of one or more different measured parameters 309, such as the compression force currently being exerted on the breast 52 and/or the current thickness of the breast 52 between the compression plate 28 and the detector 18.

In another embodiment, either alone in association with an indication 314, the ML model 302 can control the operation of the plate support mechanism 45 to cease movement of the compression plate 28 at the optimal stop point determined by the ML model 302.

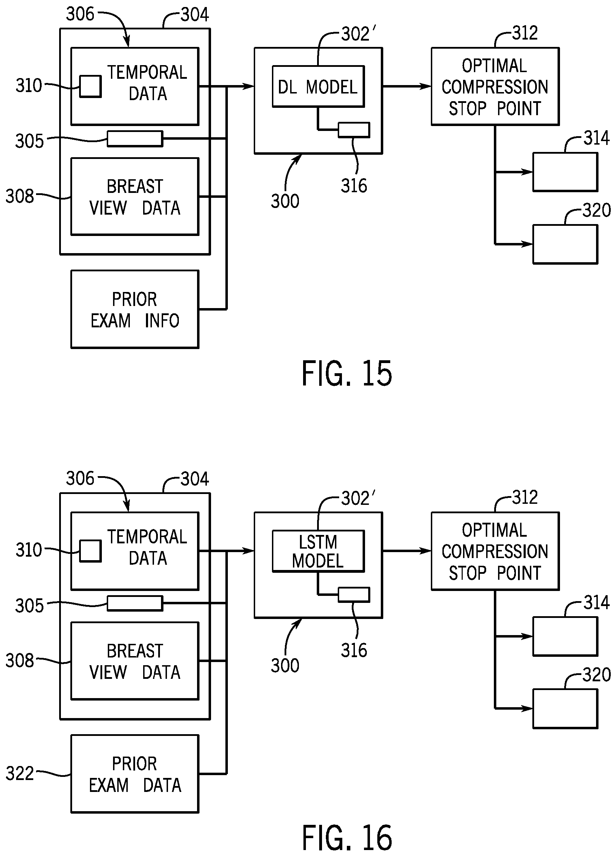

Referring now to FIG. 15, in another embodiment of the ML model 302, the ML model 302 can take the form of a deep learning (DL) model, such as a transformer neural network or recurrent neural network (RNN), and in particular a Long Short Term Memory (LSTM) network 302' particularly employed to determine and/or learn long-term dependencies in training datasets provided to the LSTM.

In one exemplary embodiment of the LSTM model 302', the LSTM model 302' operates in the manner discussed above for the ML model 302, namely, being trained on the training dataset 304/operated on the one or more measured parameters to determine and/or predict an optimal compression stop point for the compression of a breast 52 prior to performing a mammography imaging procedure, and operating and/or providing an indication 314 of when the predicted optimal stop point is reached.

Further, in still another exemplary embodiment the LSTM model 302' can include or operate in conjunction with a classifier 316. The classifier 316 operates to compare the output 312 of the LSTM model 302' on the optimal compression stop point with the current configuration of the mammography imaging system 10, e.g., the position of the compression plate 28, to classify the current configuration with regard to the optimal compression stop point. For example, the classifier 316 can provide in addition to the output 312 and indication 314, or as an alternative to the indication 314, a binary indication 320 of whether the compression has reached the optimal stop point, such that the technician and/or patient is prompted to stop the compression when the indicator 320 switches from a "NO" indicating the stop point has not been reached to a "YES" indicating the stop point has been reached.

In still a further exemplary embodiment illustrated in FIG. 16, in addition to the temporal data 306, the optional patient data 305, and the image view data 308, the training dataset 304 can include prior examination and/or image data 322, e.g., information obtained on the breast 52 being imaged in prior examinations and/or images, to obtain and/or enhance the information provided to the ML model 302 or DL/LSTM model 302' regarding the breast density and/or shape, as well as the content/tissue types, amounts and/or locations within the breast 52. This prior examination/image data 322 can be combined in the training dataset 304 to enable the ML model 302 or DL/LSTM model 302' to learn to determine or predict an optimal compression stop point in light of different tissue types present within the breast 52 and the different rates of change in the measured parameters for breasts 52 having different tissue types or content therein. As a result, when trained and provided with prior examination/image data 322 for a breast 52 to be imaged, the ML model 302 or DL/LSTM model 302' can adjust a prediction of the one or more of a series of predicted compression data points for compression of the breast 52 and/or the optimal compression stop point in light of the known content of the breast 52 being imaged.

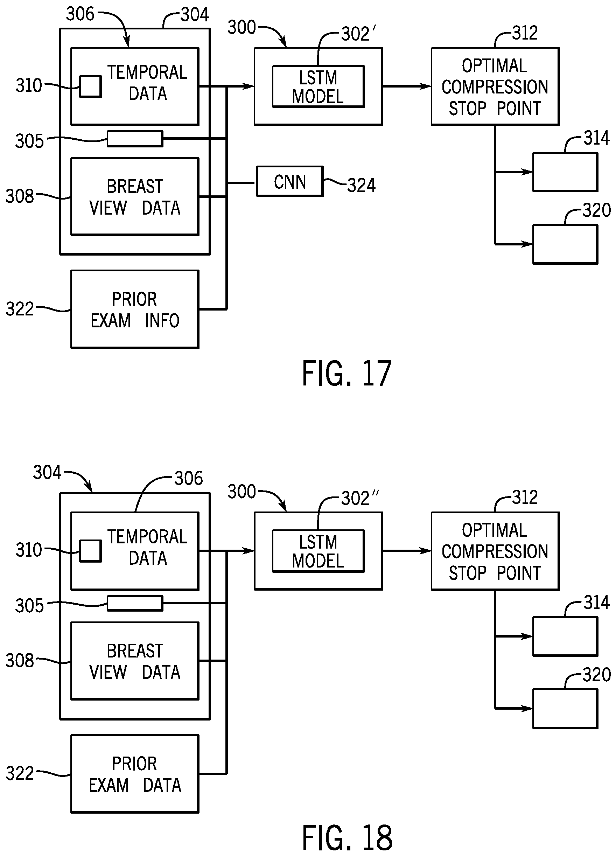

In still a further exemplary embodiment illustrated in FIG. 17, in addition to adding the prior image data 322 to the training dataset 304, the prior examination/image data 322, e.g., 1D image(s), 2D image(s), 3D images, among others, can be input to a separate convolution neural network (CNN) 324 to determine the presence and location of features of interest, e.g., scars and/or a prior determined particular pathology(ies) (e.g., large masses), within the prior examination/image data 322. The information from the CNN 324 regarding the features can be embedded with the temporal data 306 and the prior examination/image data 322 in the raining dataset 304 to further refine the training of the ML model 302 or DL/LSTM model 302' to adjust a prediction of the one or more of a series of predicted compression data points for compression of the breast 52 and/or the optimal compression stop point in light of any known features of interest within the breast 52 being imaged.

In still another exemplary embodiment, the ML model 302 can take the form of a graph neural network (GNN) 302" as illustrated in the exemplary embodiment of FIG. 18. The GNN 302" is capable of modeling complex relationships in the form of graphs that allow for the modeling of complex relationships between different variables. The graphs for the individual breast compression procedures forming the temporal data 306 and image data 308 in the training data 304 input into the GNN 302" can be formed with nodes that can represent the type of image and a variable at a given time, such as the measured parameters during an associated breast compression, and edges can represent dependencies or correlations between these variables, such how the measured parameters change in value over the breast compression procedure relative to one another and/or with regard to the type of image being obtained from image view data 308. With the analysis of the graphs forming the temporal data 306 input as a part of the training data 304 to the GNN 302" can help identify recurring patterns in series, such as cycles or trends. This is particularly useful for prediction and data analysis, such as to determine the optimal compression stop point for the compression of a breast prior to performing a mammography imaging procedure on the breast 52. Using graph theory techniques employed by the GNN 302", it is possible to analyze the temporal data 306 and image view data 308 in the training data 304 to understand how the different measured parameters interact with each other throughout the compression of a breast to reveal underlying predictive structures and hidden dynamics to increase the accuracy of the prediction made by the GNN 302" for an optimal compression stop point. Robustness and Flexibility: The graphs employed as inputs/training datasets 304 for the GNN 302" offer great flexibility for representing heterogeneous data and can be used to integrate information from different sources, such as prior examination and image data 322. This allows for a more robust and comprehensive analysis of the temporal data 304 in the training of the GNN 302" to function to predict the optimal compression stop point for a breast compression.

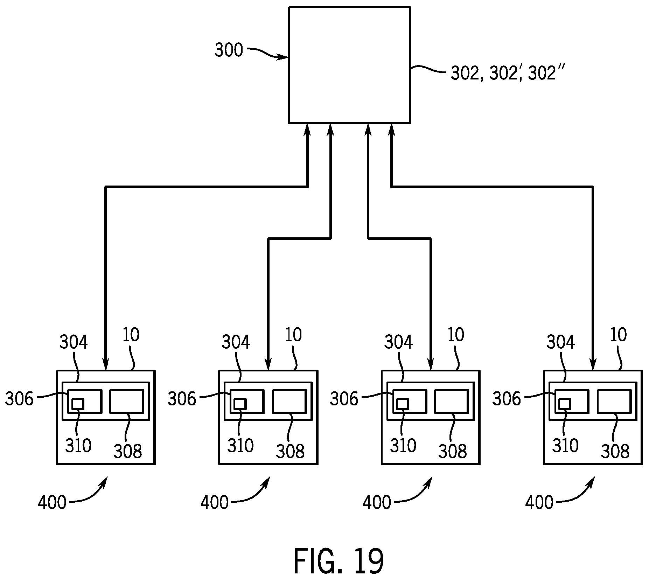

In still a further exemplary embodiment, the ML model 302, the DL/LSTM model 302" or the GNN 302" can be trained across multiple mammography imaging systems 10 in a federated manner, as shown in FIG. 19 Copies of the optimized compression module 300 in the form of the ML model 302, the DL/LSTM model 302" or the GNN 302" can be installed on mammography imaging systems 10 at various sites 400 to enable the ML model 302, the DL/LSTM model 302" or the GNN 302" to be independently trained at each site 400. Further, as the normal operation of the mammography imaging system 10 provides both the temporal data 306 and the image view data 308 for the training dataset 304, it is possible to train the ML model 302, the DL/LSTM model 302" or the GNN 302" at each site 400, or at a single site 400, in a continuous manner with each breast compression and associated ground truth 310 performed by the mammography imaging system 10 creating an addition to the training dataset 304 employed to train the ML model 302, the DL/LSTM model 302" or the GNN 302".

In one exemplary embodiment, after the ML model 302, the DL/LSTM model 302" or the GNN 302" is trained at a particular site 400, with the training dataset 304 employed being representative of the demographics and other relevant data for the patients imaged at the site 400. As a result, the trained ML model 302, the DL/LSTM model 302" or the GNN 302" is tailored for use in predicting the compression curves and optimal compression stop points for the population in the region around the site 400.

Alternatively, in order to train the ML model 302, the DL/LSTM model 302" or the GNN 302" in a more robust manner applicable to a wider demographic scope or range of patients, after each of the ML model 302, the DL/LSTM model 302" or the GNN 302" being trained at the various sites 400 have reached a predetermined confidence level, the ML model 302, the DL/LSTM model 302" or the GNN 302" can be recombined into a single ML model 302, the DL/LSTM model 302" or the GNN 302" to form the optimized compression module 300 for copying and/or installation on a mammography imaging system 10 to determine optimal compression stop points for mammography imaging procedures in the manner described above.

Finally, it is also to be understood that the system 10 may include the necessary electronics, software, memory, storage, databases, firmware, logic/state machines, microprocessors, communication links, displays or other visual or audio user interfaces, printing devices, and any other input/output interfaces to perform the functions described herein and/or to achieve the results described herein. For example, as previously mentioned, the system may include at least one processor and system memory/data storage structures, which may include random access memory (RAM) and read-only memory (ROM). The at least one processor of the system 10 may include one or more conventional microprocessors and one or more supplementary co-processors such as math co-processors or the like. The data storage structures discussed herein may include an appropriate combination of magnetic, optical and/or semiconductor memory, and may include, for example, RAM, ROM, flash drive, an optical disc such as a compact disc and/or a hard disk or drive.

Additionally, a software application that adapts the controller to perform the methods disclosed herein may be read into a main memory of the at least one processor from a computer-readable medium. The term "computer-readable medium", as used herein, refers to any medium that provides or participates in providing instructions to the at least one processor of the system 10 (or any other processor of a device described herein) for execution. Such a medium may take many forms, including but not limited to, non-volatile media and volatile media. Non-volatile media include, for example, optical, magnetic, or opto-magnetic disks, such as memory. Volatile media include dynamic random access memory (DRAM), which typically constitutes the main memory. Common forms of computer-readable media include, for example, a floppy disk, a flexible disk, hard disk, magnetic tape, any other magnetic medium, a CD-ROM, DVD, any other optical medium, a RAM, a PROM, an EPROM or EEPROM (electronically erasable programmable read-only memory), a FLASH-EEPROM, any other memory chip or cartridge, or any other medium from which a computer can read.

While in embodiments, the execution of sequences of instructions in the software application causes at least one processor to perform the methods/processes described herein, hard-wired circuitry may be used in place of, or in combination with, software instructions for implementation of the methods/processes of the present invention. Therefore, embodiments of the present invention are not limited to any specific combination of hardware and/or software.

It is understood that the aforementioned compositions, apparatuses and methods of this disclosure are not limited to the particular embodiments and methodology, as these may vary. It is also understood that the terminology used herein is for the purpose of describing particular exemplary embodiments only, and is not intended to limit the scope of the present disclosure which will be limited only by the appended claims.

## Claims

1. A method for determining an optimal compression stop point prior to performing a mammography imaging procedure, the method comprising the steps of:
a. providing a mammography imaging system comprising:
i. a gantry disposed movably disposed on a support surface and including a radiation source, a detector alignable with the radiation source, the detector having a surface on which a breast to be imaged is adapted to be positioned, and a compression plate disposed on the gantry and movable with respect to the radiation source and the detector;
ii. a controller operably connected to the gantry to control the operation of the radiation source and detector to generate image data in an imaging procedure performed by the imaging system, the controller including a central processing unit and interconnected database for processing the image data from the detector to create images, a display operably connected to the controller for presenting information to a user, and a user interface operably connected to the controller to enable user input to the controller; and
iii. an optimized compression module operably connected to the controller, the optimized compression module including at least one sensing device operable to measure operating parameters of the mammography imaging system and generate compression data;
b. positioning the breast on the surface between the radiation source and the detector;
c. operating the at least one sensing device to measure parameters and generate the compression data,
d. comparing the compression data to one or more of a series of predicted compression data points for compression of the breast and an optimal compression stop point determined by the optimized compression module based on the measured parameters; and
e. optionally adjusting the operation of one or more components of the mammography imaging system based on the comparison of the compression data to the one or more of a series of predicted compression data points for compression of the breast and the optimal compression stop point.

2. The method of claim 1, wherein the optimized compression module is a machine learning (ML) module.

3. The method of claim 1, wherein the at least one sensing device is a time-of-flight (ToF) sensor.

4. The method of claim 1, wherein the step of comparing the compression data to one or more of a series of predicted compression data points for compression of the breast and an optimal compression stop point determined by the optimized compression module based on the measured parameters further comprises comparing the compression data to one or more of a series of predicted compression data points for compression of the breast and an optimal compression stop point determined by the optimized compression module based on the measured parameters associated with corresponding image views.

5. A mammography imaging system comprising:
a. a gantry including radiation source, a detector alignable with the radiation source, and a compression plate moveable relative to the detector to secure a patient breast therebetween;
b. a controller operably connected to the gantry to control the operation of the radiation source and detector to generate image data, and to control the operation of the biopsy device in an interventional/biopsy mode for the mammography system, the controller including a central processing unit and interconnected database for processing the image data from the detector, a display operably connected to the controller for presenting information to a user, and a user interface operably connected to the controller to enable user input to the controller; and
c. an optimized compression module operably connected to the controller, the optimized compression module including at least one sensing device and operable to generate compression data regarding the breast disposed on the detector and to guide the movement of the compression plate to compress the breast on the detector.

6. The mammography imaging system of claim 4, wherein the at least one sensing device is mounted to the gantry on or adjacent the detector, on or adjacent the radiation source, on or adjacent the compression plate, or combinations thereof, and is operable to determine positions of the compression plate, the breast and the detector relative to each other.

7. The mammography imaging system of claim 5, wherein the controller is configured to control the movement of the compression plate in response to the positions of the compression plate, the breast and the detector relative to each other.

8. The mammography imaging system of claim 4, wherein the optimized compression module is configured to receive the data for the positions of the compression plate, the breast and the detector relative to each other compute at least one of a breast shape, a 3D breast image, a speed of movement of the compression plate, a pressure exerted on the breast, and combinations thereof for predicting one or more of a series of predicted compression data points for compression of the breast and an optimal compression stop point.

9. The mammography imaging system of claim 7, wherein the optimized compression module provides an output from the comparison of the current force exerted, breast thickness, breast shape, 3D breast image, speed of movement of the compression plate, pressure exerted on the breast, and combinations thereof with the one or more of a series of predicted compression data points for compression of the breast and the optimal compression stop point.

10. The mammography imaging system of claim 8, wherein the output from the optimized compression module is an indicator, feedback of the comparison of one of current force exerted, breast thickness, breast shape, 3D breast image, speed of movement of the compression plate, pressure exerted on the breast, and combinations thereof with the one or more of a series of predicted compression data points for compression of the breast and the optimal compression stop point, control of movement of the compression plate, and combinations thereof.

11. The mammography imaging system of claim 4, wherein the at least one sensing device is a ToF sensing device.

12. The mammography imaging system of claim 4, wherein the optimized compression module is a machine learning (ML) module trained to output the predicted optimized compression stop point utilizing a training dataset comprising:
a. temporal data obtained from breast compressions performed prior to mammography imaging procedures including parameters recorded during the compression of a breast to determine a compression force exerted on of the breast;
b. image view data identifying an image view associated with each breast compression; and
c. a ground truth regarding an actual compression stop point for each breast compression.

13. The mammography imaging system of claim 12, wherein the ground truth is a technologist-initiated compression stop point, a patient-initiated compression stop point, or combinations thereof.
